# EUROPEAN PATENT APPLICATION

(11) **EP 4 439 573 A1**
(43) Date of publication of application: **02.10.2024**
(21) Application number: 23164665.4
(22) Date of filing: 28.03.2023
(51) Int. Cl.: G16H 10/60, G06F 12/0875, G06F 16/172, H04L 67/568

(54) **SELECTIVE CACHING OF MEDICAL DATA**

(71) Applicant: Koninklijke Philips N.V., 5656 AG Eindhoven (NL)
(72) Inventor: BULUT, Murtaza, 5656 AG Eindhoven (NL); BOUTS, Mark Jacobus Rosalie Joseph, 5656 AG Eindhoven (NL); HUIJBREGTS, Laurentia Johanna, 5656 AG Eindhoven (NL)
(74) Representative: Philips Intellectual Property & Standards

(57) **Abstract**

A mechanism for selective caching of medical data. A use or purpose of the medical data is obtained. The medical data is selectively cached responsive to the use or purpose for the medical data.

## Description

### FIELD OF THE INVENTION

The present invention relates to the field of medical data management, and in particular to the selective caching of medical data.

### BACKGROUND OF THE INVENTION

There are a number of tasks or procedures that process medical data in order to guide a clinician in making a clinical decision. It is common for medical data to be stored in an electronic medical data store such as an electronic medical record (EMR), from which it needs to be extracted for any subsequent processing, e.g., for providing protocol execution related guidance.

However, extraction of medical data from an electronic medical data store is not a straightforward task. It is common for electronic medical data stores to be stored at a remote location to where the stored medical data is to be used. Accessing and/or updating the electronic medical data stores can therefore be a computational expensive task, e.g., requiring significant bandwidth, and/or incur delay due to latency. Moreover, an electronic medical data store is not usually a high-fidelity device, but rather a mere data repository. Thus, data availability, latency and reliability may be problematic and fall outside the demands of any processes or protocols that need to use the stored medical data, such as protocol guidance.

One approach to overcoming at least these issues is to extract and store a local copy of the electronic medical data store in a memory cache. Accessing the local copy avoids the need to use computationally expensive communication channels and reduces any latency in accessing the relevant medical data.

There is an ongoing desire to improve the efficiency of extracting and/or using medical data available in an electronic medical data store.

### SUMMARY OF THE INVENTION

The invention is defined by the claims.

According to examples in accordance with an aspect of the invention, there is provided a computer-implemented method for selective caching of medical data, contained in an electronic medical data store, in a memory cache that is remote from the electronic medical data store.

The computer-implemented method comprises: receiving an indication of an intended purpose or use for the medical data; determining whether or not the medical data is to be cached responsive to the received indication of the intended purpose or use for the medical data; and storing the medical data in the memory cache only when it is determined that the medical data is to be cached.

The present disclosure proposes a technique for selective caching of medical data, available in an electronic medical data store such as an electronic medical record (EMR), responsive to the intended purpose or use for the medical data.

It has been herein recognized that retrieval of a full copy of the electronic medical data store may require significant bandwidth and would be computationally inefficient if only a small part of the electronic medical data store is subsequently actually required for processing. By using the proposed selective caching technique, only the relevant parts of the medical data are cached or stored. In particular, using the proposed selective caching technique, data is only cached based on the intended purpose for the medical data, which can avoid or reduce the caching of unneeded or unnecessary data.

As previously mentioned, caching of data is useful for as an electronic medical data store is not usually a high-fidelity device, meaning that data availability, latency and reliability of the data store may be problematic and fall outside the demands of any processes or protocols that need to use the stored medical data, such as protocol guidance. By caching the relevant data, these issues can be avoided or mitigated.

The medical data may, for instance, be only a part (i.e., not all) of the total medical data (for a particular subject) stored in the electronic medical data store.

Preferably, the electronic medical data store is stored at a remote location to the system carrying out the proposed method. In particular, a latency between the system storing the electronic medical data store and the system performing the proposed method is preferably greater than 100ms, e.g., greater than 250ms. Thus, a latency between the memory cache and the electronic medical data store is preferably greater than 100ms, e.g., greater than 250ms. Proposed approaches are particularly advantageous when there is a large latency, which would significantly affect the performance of methods/programs/protocols that make use of the medical data.

In some examples, the step of storing the medical data further comprises storing, in the memory cache, information suitable for identifying an original location of the medical data in the electronic medical data store for facilitating automated updating of the medical data in the electronic medical data store with a modified version of the cached medical data.

This embodiment facilitates ease of updating the electronic medical data store. In particular, automated updating of the medical data in the original electronic medical data store (e.g., an EMR) can be achieved. The information may, for instance, facilitate mapping of the cached version of the medical data to a location in the electronic medical data store. The information may, for instance, be in the form of a pointer.

As another example, the information may be in form of the structure or format of the cached medical data. The precise structure or format of medical data may be unique to a particular location or region of the electronic medical data store. By storing the medical data in a same structure or format, it is possible to identify the corresponding location in the electronic medical data store.

As yet another example, the information may comprise supplementary data that is linked to the medical data in the electronic medical data store, such as dependency, links and/or connection information.

In this way, the caching may be performed under the assumption that there is an intention to update the medical data and push it back to the source electronic medical data store (e.g., EMR). Accordingly, it is proposed to store structure or pointer information for updating the source electronic medical data store (e.g., EMR).

The information suitable for identifying the original location of the medical data may comprises metadata of the medical data. Metadata also provides identifying information that facilitate identification of the original location of the medical data in the electronic medical data store.

The step of storing the medical data may comprise storing the medical data, in the memory cache, in a same data structure as the medical data is stored in the electronic medical data store.

The indication of an intended purpose or use for the medical data may comprise a trigger signal generated during a computer-implemented protocol or program that indicates that the medical data will be required during later processing performed by the computer-implemented protocol or program.

It is recognized that a computer-implemented protocol or program for processing medical data can have a plurality of different paths or sub-processes that are performed, e.g., depending upon the information contained in the medical data. By using a trigger signal, the relevant medical data can be retrieved and cached in advance of its need for processing by the protocol/program (e.g., but not before it is known that it will be required). This improves the overall performance of the protocol or program, by reducing a latency required to extract the medical data from the electronic medical data store.

The step of determining whether or not the medical data is to be cached optionally comprises: processing the indication of an intended purpose or use to predict an access frequency of the medical data during the intended purpose or use; and determining whether or not the medical data is to be cached further responsive to the predicted access frequency of the medical data.

It has been recognized that if data needs to be repeatedly accessed or used for its intended purpose, that this would have previously required significant numbers of access requests to the electronic medical data store. By performing selective caching based on the number of predicted accesses (e.g., predicted access frequency), medical data that is in high demand can be cached, whilst data that is in less demand can avoid being cached (e.g., to save on memory cache space). This approach can therefore prioritize the caching of in-demand medical data, i.e., medical data that is to be retrieved frequently.

The step of determining whether or not the medical data is to be cached optionally comprises processing the indication of an/the intended purpose or use to predict a time of access of the medical data during the intended purpose or use; and determining whether or not the medical data is to be cached further responsive to the predicted time of access.

It has been recognized that if the medical data is not required for some time, then caching the data may be an inefficient use of cache memory. Thus, it may be possible to selectively cache based on a predicted time of access. Of course, this process may comprise delaying a caching of the medical data responsive to the predicted time of access, e.g., until closer to the predicted time of access.

The intended purpose or use for the medical data may be an intended protocol under which the medical data is to be processed and/or an intended portion of a protocol under which the medical data is to be processed.

The step of determining whether or not the medical data is to be cached may comprise: processing the indication of an intended purpose or use to predict a size of the medical data; and determining whether or not the medical data is to be cached further responsive to the predicted size of the medical data.

There is a desire to reduce the number of access requests to an electronic medical data store for large amounts of data, as this requires significant bandwidth and processing power to communicate. By using the predicted size of the medical data in determining whether to cache the medical data, then medical data of a large size can be cached to reduce the number of access requests to the electronic medical data store, such as an EMR.

It is possible to use the intended purpose to predict the size, e.g., as the intended purpose will change responsive to the size of the data to be processed and/or otherwise be indicative of the size of the medical data. For instance, if the intended purpose is to use the medical data as an input feature for a machine-learning method, this can indicate that the medical data is relatively small, whereas if the intended purpose is to search for frequency of key words in the medical data, then this can indicate that the medical data is relatively large.

The step of determining whether or not the medical data is to be cached may be further responsive to the bandwidth of the communication with the medical data storage. For instance, in some cases, if it is expected that there will be limited bandwidth available, it may be preferable to cache the data, e.g., even if the cached data has a small size.

The step of determining whether or not the medical data is to be cached may be further responsive to the content of the medical data. Some forms of medical data would benefit from being cached more than others. For instance, a medical history of a subject is less likely to change in a near future than one or more monitored signs of the subject.

In some examples, the step of determining whether or not the medical data is to be cached comprises determining that the medical data is not to be cached responsive to the content of the medical data being data that is known to change quickly, such as physiological parameters of the subject.

In some examples, the step of determining whether or not the medical data is to be cached comprises determining that the medical data is not to be cached responsive to the content of the medical data being data that is manually defined by a human.

Preferably, the computer-implemented method is performed by a system that is remote from the electronic medical data store.

In other examples, said computer-implemented method is performed by a medical record system that comprises the electronic medical data store. In this way, the medical record system itself can determine whether the medical data is to be cached in the memory cache, e.g., which is remote from the medical record system. This offloads the caching determination procedure from the caching system, and could make use of superior processing resource at the medical record system.

The method may further comprise updating the cached medical data responsive to one or more input signals; and updating, responsive to a user input, the medical data stored in the electronic medical data store with the updated cached medical data.

In this way, a user is able to control whether the medical data in the electronic medical data store is updated (e.g., replaced or supplemented) with modified cached medical data. This provides a mechanism for secure conflict resolution between the cached medical data and the medical data in the electronic medical data store, reducing a risk of error or undesirable automated overwriting of existing medical records.

There is also proposed a computer-implemented method for retrieving medical data available for retrieval in an electronic medical data store, the computer-implemented method comprising: obtaining a request for the medical data; determining whether or not the medical data is stored in a memory cache; responsive to determining that the medical data is stored in the memory cache, retrieving the medical data from the memory cache; and responsive to determining that the medical data is not stored in the memory cache: retrieving the medical data from the electronic medical data store and performing any previously described computer-implemented method for the selective caching of the retrieved medical data.

There is also proposed a computer program product comprising computer program code means which, when executed on a computing device having a processing system, cause the processing system to perform all of the steps of any herein disclosed method.

There is also proposed a caching system for selective caching of medical data, contained in an electronic medical data store, the caching system comprising: a memory cache remote to the electronic medical data store; and a processing system configured to: receive an indication of an intended purpose or use for the medical data; determine whether or not the medical data is to be cached responsive to the received indication of the intended purpose or use for the medical data; and store the medical data in the memory cache only when it is determined that the medical data is to be cached.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a system in which embodiments can be employed;
Fig. 2 is a flowchart illustrating a method;
Fig. 3 is a flowchart illustrating another method;
Fig. 4 is a flowchart illustrating a further method; and
Fig. 5 is a flowchart illustrating yet another method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for selective caching of medical data. A use or purpose of the medical data is obtained. The medical data is selectively cached responsive to the use or purpose for the medical data.

Embodiments are based on the realization that caching of medical data is a computationally expensive task, but that it might be beneficial to do so in order to reduce a number of access requests to an electronic medical data store or avoid a latency in accessing the medical data). In particular, the present disclosure recognizes that the purpose or intended use for medical data will affect or otherwise influence whether it is computationally better to cache the medical data or to not cache the medical data. This is because the use/purpose will determine how long until the medical data will be used (and therefore whether the cached version will be inaccurate or out of date by the time it is used), how often the medical data will be used (and therefore how many requests to the medical record can be avoided by caching) and/or the size of the medical data (and therefore how long it will take to retrieve the medical data from the medical record when it is required). All of these factors can be taken into account across different embodiments of the invention, depending upon the specific use-case scenario and/or implementation environment.

Embodiments can be employed in any system that involves a processing device that desires medical data stored in an electronic medical data store for proceeding or performing some other task, e.g., for the generation of clinical guidance or advice.

The present disclosure relates to the field of memory management, and particularly to the selective caching of data. Embodiments may make use of well-known functions in this field, non-exhaustively including approaches for: caching data, using cached data, monitoring a cache, monitoring for updates to a cache, retrieving data from a memory or cache and so on. These techniques follow well established and widely available procedures, and are not described in detail for the sake of conciseness. Rather, for the sake of explanative clarity, the present disclosure focuses upon the distinguishing differences and specific usage of such widely known functions to achieve new advantages.

In the context of the present application, a protocol that process medical data is any suitable medical protocol that makes use of medical data to make a clinically-relevant recommendation, guidance or output. For instance, a protocol may be a protocol for determining a predicted likelihood of a subject having a particular pathology or condition. A protocol therefore sets out or defines a series of steps that are to be performed that involve processing of medical data to arrive at some output.

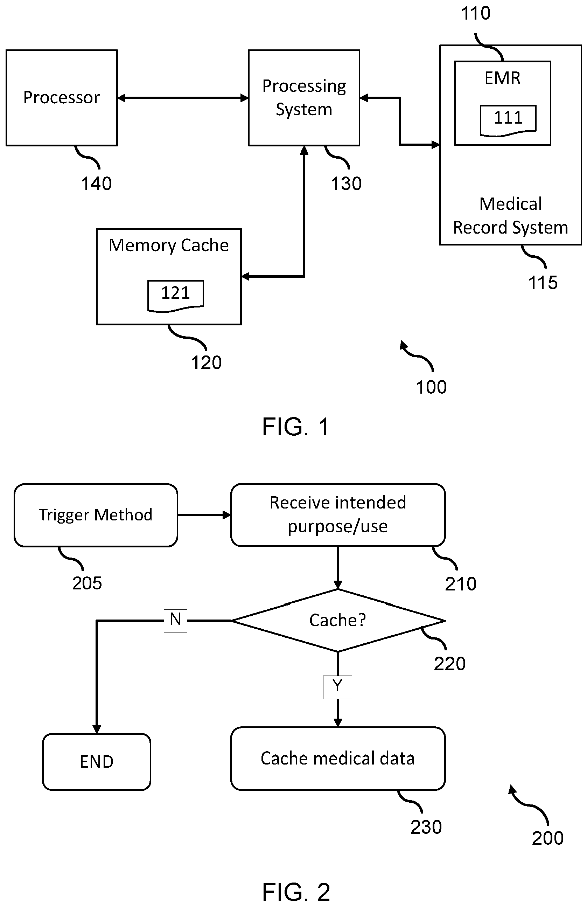

Fig. 1 illustrates a system 100 in which embodiments of the invention can be employed.

The system 100 comprises an electronic medical data store 110, a memory cache 120, a processing system 130 and a processor 140. The memory cache 120 and the processor 140 are remote from the electronic medical data store 110, e.g., located at a different site, e.g., such that there is a latency of at least 10ms (or more preferably at least 50ms, more preferably at least 100ms) for communications between the two sites.

Put another way, the electronic medical data store 110 is external to the memory cache 120 and the processor 140. The processing system 130 may act as the intermediary between the processor 140 and the memory cache 120 and/or electronic medical data store.

The medical record system 115 and/or the memory cache 120 may comprise any suitable volatile and/or non-volatile computer memory such as RAM, PROM, EPROM, and EEPROM. The memory cache 120 is preferably a high-speed memory such as RAM or the like. In particular examples, the memory cache may be an inbuilt cache memory of the processor 140.

The electronic medical data store 110 may form part of a larger medical record system 115, e.g., be a data structure stored in the larger medical record system 115. The electronic medical data store 110 may contain data for a single subject or individual. In particular examples, the electronic medical data store 110 may be an electronic medical record for a subject, although other forms of medical data storage are known.

As a general working principle, the processor 140 desires access to some medical data 111 stored in the electronic medical data store 110 for performing some task (e.g., displaying of the medical data or processing of the medical data). Historically, the processor 140 would direct an access request to the electronic medical data store 110 to retrieve the medical data 111. This naive approach can be computationally expensive (due to the need to transmit over a resource expensive communication channel such as the internet or a LAN connection) and involve significant latency, which affects the speed and efficiency of the task performed by the processor 140.

One approach for reducing the computational expense and/or latency of accessing the medical data 111 is to cache the medical record 110 in the memory cache 120. Thus, the memory cache 120 may store a cached version 121 of the electronic medical data store. Future accessing of the medical data 111 by the processor 140 can then simply retrieve the required medical data from the cached version 121 of the electronic medical data store, for reduced expense and latency. The retrieval and caching of the electronic medical data store may be performed by the processing system 130. Thus, the processing system may control the caching of the medical data 111 in the memory cache 120.

The present disclosure recognizes that certain (predictable) processes will not require access to the entire medical data store (e.g., EMR) and that not all data of a medical data store is suited for being cached in this way. It is proposed to ascertain or determine an intended use for medical data and perform selective caching of medical data (from the electronic medical data store) based on the intended use.

Thus, only a portion of the electronic medical data store needs to be cached in the memory cache 120 based on an intended use (and other optional characteristics) of the medical data.

The proposed approach provides a more computationally and time efficient mechanism for providing a processor with medical data for performing a task. In particular, the proposed technique improves a distribution of data between a memory cache and an electronic medical data store for facilitating a balance between faster and more efficient processing of medical data whilst retaining good accuracy in the performance of a task.

Whilst this application is directed towards the principle for selective caching of medical data, it will be appreciated that the disclosed concepts can apply to any other form of data or data type stored in an electronic medical data store. This may include financial data, scheduling information, operational data, medication data (i.e., information identifying the content and/or possible effects of medication), administration data, user interface related data (e.g. settings or graphics data), user interaction related data (e.g. approvals required, rights and/or permissions).

Medical data is considered to be medical information usable (e.g., by a suitably qualified or experienced clinician) to determine or analyze a medical condition and/or pathology of a subject associated with the medical data. This term is well known in the art.

Fig. 2 is a flowchart illustrating a method 200 according to a basic embodiment of the invention. The method 200 is carried out by a processing system, which is effectively a computer, such that the method is a computer-implemented method. In particular, with reference to Fig. 1, the method 200 may be performed by the processing system 130.

As previously explained, the method 200 is configured for selective caching of medical data, contained in an electronic medical data store, in a memory cache that is remote from the electronic medical data store.

The method 200 comprises a step 210 of receiving an indication of an intended purpose or use for the medical data. The indication may, for instance, be provided by an individual or human wishing to access or make use of the medical data and/or by a computer program or protocol that intends to access or make use of the medical data. The intended purpose or use for the medical data may therefore be an intended protocol under which the medical data is to be processed.

The indication may be direct or indirect. For instance, the individual may be able to indicate that specific data is needed (from the electronic medical data store) for a specific purpose (direct), but may alternatively be able to indicate a specific medical procedure that is to be applied or used, from which the method or system may automatically determine the necessary medical data for retrieval from the electronic medical data store that is linked to the specific medical procedure (indirect).

The method 200 then performs a step 220 of determining whether or not the medical data is to be cached responsive to the received indication of the intended purpose or use for the medical data.

The method 200 then comprises a step 230 of storing the medical data in the memory cache only when it is determined that the medical data is to be cached. In this way, selective caching of medical data is performed responsive to the intended purpose or use for the medical data, e.g., the purpose for which the medical data is to be used by a processor 140 in performing a task that makes use of the medical data.

In preferred examples, the method 200 is performed before the medical data is needed or required for use by a processor that performs a task using the medical data. Thus, predictive, selective caching of data can be performed.

As previously mentioned, the intended purpose or use for the medical data may include an indication of an intended protocol or procedure under which the medical data is to be processed. This facilitates identification of useful information for determining whether it is computationally worthwhile to cache the medical data, e.g., for future use.

In one example, the intended protocol is defined in a document, flowchart or piece of code. The intended protocol may, for instance, analyzed to determine one or more properties of the medical data that is to be used by the medical protocol, e.g., the time before accessing, the amount, the type and/or the frequency of access of medical data. If the amount, frequency, and update regularity is greater than some predefined thresholds (or the time before accessing is below some threshold), then the medical data may be cached in the memory cache in step 230. Otherwise, when required, the data can be retrieved from the electronic medical data store.

Thresholds can be set according to the historical values of previous data extractions (e.g., median values, or 25% quantile can be used). The thresholds can be dynamically adapted, and can be updated based on a particular setting of the processor or processing system.

For instance, initial thresholds can be set as described above. Subsequently, the usage or needs of the processor, processor system and/or memory cache may be monitored and the thresholds can be updated accordingly. Typically, the thresholds would be updated so that more data is cached over time (so that the processor works better, faster, and more accurately).

In some examples, for the purposes of step 210, the indication of an intended purpose or use for the medical data comprises a trigger signal generated during a computer-implemented protocol or program that indicates that the medical data will be required during later processing performed by the computer-implemented protocol or program.

In this way, the trigger signal may indicate a predicted future use of the medical data. A trigger signal may be generated by a processor performing a predetermined protocol or program, e.g., when a certain function is executed or performed. Such a function may, for instance, only be performed during a procedure in which the medical data will be required in future, e.g., in future steps of the procedure.

This embodiment appreciates that certain procedures performed by a processor or other device have predictable needs for certain portions or extracts of medical data. For instance, an automated subject/patient analysis process may be a multi-step process (e.g., a decision tree) that sequentially makes use of different instances of medical data. Some parts of medical data may be required during some instances of executing the process but not during others. A trigger signal may indicate when it is known that a certain instance of medical data is (likely to be) needed in future steps.

Put yet another away, the trigger signal may indicate which part of a known protocol or procedure is to be followed. This indicated part may make use of known medical data, such that an intended use for the medical data is indicated. This information is usable to determine or derive whether caching the medical data (to be used in the future part of the known protocol or procedure) is worthwhile. The decision on whether it is worthwhile to cache the medical data will depend upon the specific implementation, and embodiments are not restricted to any specific decision or choice as to when data will be cached. Rather, embodiments recognize that it would be advantageous to make the decision on whether to cache the medical data based on a future or intended use for said medical data.

It will be appreciated that not all instances of following a same protocol or procedure may necessitate use of a same set of medical data. For instance, if the protocol makes use of a decision tree, different branches of the decision tree may make use of different sets of medical data. When a certain decision is made in the decision tree, such that a future set of required medical data is known or predictable, then the trigger signal may be produced identifying the medical data and the use or purpose of the medical data. This same conceptual understanding applies to other forms of protocols or processes, such as if-this-then-that processes or the like.

Put another way, the trigger signal can act as an indicator that the future task of the medical data is that the medical data will be used in processing by a program or protocol. This embodiment facilitates predictive (but selective) retrieval and storage of relevant medical data for performing a task.

However, other suitable forms for an indication of an intended purpose or use will be apparent to the skilled person, such as an indication provided by a user (via a user interface), by a processor (e.g., following an automated analysis mechanism) or by a schedule (e.g., if there is a schedule for performing a certain task on medical data at certain times). Other suitable examples will also be apparent to the skilled person.

In one example, step 220 comprises processing the indication of an intended purpose or use to predict an access frequency of the medical data during the intended purpose or use; and determining whether or not the medical data is to be cached further responsive to the predicted access frequency of the medical data.

It is recognized that a future use for the medical data will influence how often the medical data is to be accessed or required in carrying out the future use. By selectively caching medical data based on its access frequency, a balance can be struck between the computational expense of caching data and retrieving data.

For instance, if medical data is to be used multiple times during its future use, it would be preferred to cache that data to avoid repeated access requests to the electronic medical data store. On the other hand, if the medical data is to be used only once in its intended purpose or use, then a latency of retrieving the medical data from the electronic medical data record may be acceptable (as access would be required in any event, e.g., even to cache the data).

If the purpose of the data is to be used in a medical protocol or program that makes use of the data, access frequency can be easily predicted based on the future steps of the protocol or program (which defines when the medical data is needed). As an example, a trigger signal may indicate a path of a computer program or protocol that will be carried out in the future, which can indicate how often the medical data will need to be accessed.

Other suitable examples will be known to the skilled person. For instance, a schedule may itself define that the purpose or use of the medical data is to be processed periodically, with the periodicity defining the access frequency.

In some examples, the step 220 of determining whether or not the medical data is to be cached comprises processing the indication of an intended purpose or use to predict a time of access (or time before access) of the medical data during the intended purpose or use; and determining whether or not the medical data is to be cached further responsive to the predicted time of access (or time before access).

The time of access can be defined in terms of number of steps of a procedure that makes use of the medical data and/or an elapsed time (e.g., measurable in seconds). This approach recognizes that a purpose or use of medical data can define when the medical data is actually to be used. This approach can avoid or reduce early caching of the medical data before it is needed, thereby providing more efficient use of the memory cache.

In some examples, the step 220 of determining whether or not the medical data is to be cached comprises processing the indication of an intended purpose or use to predict a size of the medical data; and determining whether or not the medical data is to be cached further responsive to the predicted size of the medical data.

This approach recognizes that a use for medical data is indicative of the size of the medical data. For instance, certain protocols may use medical data in an equation (which indicates the data is of a relatively small size - e.g., a single floating value), whereas others may perform an iterative search in medical data (which indicates the data is of a relatively large size). As another example, a protocol or program may define the size of data to be retrieved, e.g., by defining the size of an array, which will indicate a predicted size of the medical data. It will be appreciated that these are non-exhaustive examples that merely serve to exemplify how it is possible to derive or predict a size of the medical data based on its intended use or purpose, and the skilled person would be readily capable of determining other approaches dependent upon the specific use case scenario.

This embodiment recognizes that it may be beneficial to use the size of medical data to influence the decision of whether or not the medical data is to be cached. For instance, if the medical data is extremely large, then it may be preferred to cache the medical data to avoid a long latency in accessing the medical data. Alternative embodiments may prefer to not cache the medical data if it is extremely large, to avoid overloading the cache. It is appreciated that the precise mechanism will depend upon the desires of an operator of the overall system.

Of course, a combination of (predicted) access frequency and size can be used to make the decision on whether or not to cache the medical data in step 220. For instance, if the medical data has a relatively large size (e.g., larger than a threshold size) and will be accessed more than a threshold number of times, then it may be advantageous to cache the medical data to avoid repeatedly accesses of large medical data. Conversely, if the medical data has a relatively small size (but will be accessed a same number of times as the relatively large data) then it may be acceptable to still access this medical data directly from the medical record. The value of the threshold number of times may, for instance, therefore depend upon the (predicted) size of the medical data to be accessed.

It will be appreciated that the precise values of the thresholds mentioned above, if used, will depend upon the use-case scenario and can vary based on implementation detail without departing from the underlying inventive recognition.

In some examples, the step 220 of determining whether or not the medical data is to be cached is (further) responsive to the content of the medical data, i.e., metadata regarding the information contained in the medical data identifying its properties or the like.

The content of the medical data can, for instance, be known in advance. This is because a task/protocol/process that makes use of the medical data will define the content of the medical data that it wishes to use, e.g., as it is programmed to retrieve or use a known content of medical data. For instance, a protocol may wish to access a patient history, such that it is known that the medical data to be accessed contains a patient history.

This embodiment appreciates that some medical data to be accessed is more suited for caching than others, e.g., is less likely to change or be updated after caching. For instance, medical data that is regularly updated in the electronic medical data store is less suited for caching, as the cached version may become inaccurate when it is later used, reducing a reliability of the task that uses the medical data.

In some examples, the step of determining whether or not the medical data is to be cached comprises determining that the medical data is not to be cached responsive to the content of the medical data being data that is manually defined by a human. Human-defined data is more likely to be updated in the electronic medical data store, e.g., compared to automatically stored data that may represent historic values of the subject.

The steps 210-230 may be performed, for instance, responsive to a trigger. Thus, the method may comprise a step 205 of receiving a trigger and performing the steps 210-230 responsive to the trigger.

This approach recognizes that the decision on whether or not to cache certain medical data can be made at any stage, e.g., any stage during a protocol or procedure that makes use of the medical data. For instance, the decision could be made at the beginning of a protocol or procedure, but it can be also made later during the procedure.

The trigger may be the receipt of a trigger signal, e.g., as used in a previously described embodiment of step 210. However, there may be alternative trigger signals for initiating the performance of the method. One example trigger is the value(s) of one or more pre-determined parameters of the processor (e.g., processor load, calculation time, data load time and so on) breaching one or more predetermined thresholds, which may be user-defined or protocol-defined. Another example trigger is a user input trigger. Another example trigger is an indication from the memory cache of free space. Yet another example, later described, is a request to access the medical data (e.g., from the processor). Other suitable triggers will be readily apparent to the skilled person.

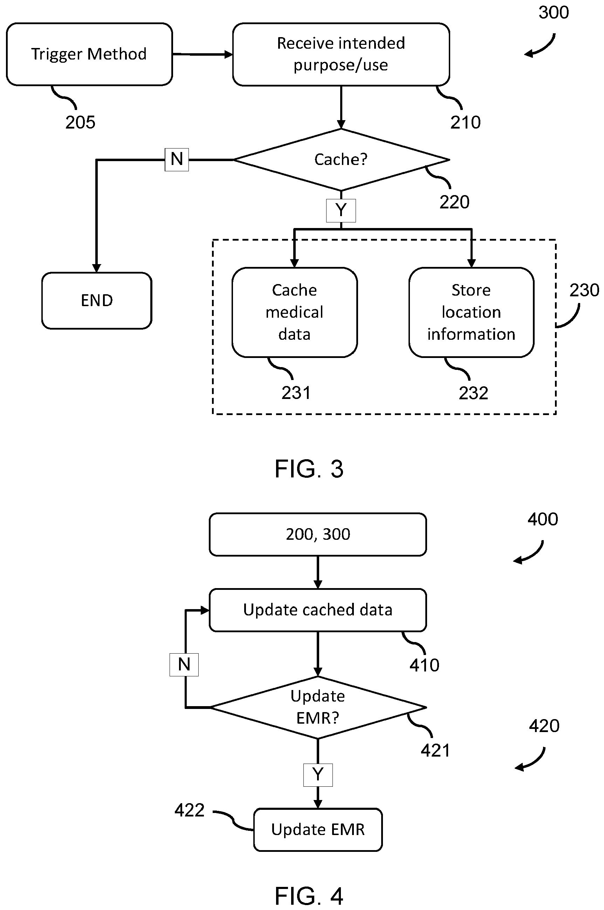

Fig. 3 is a flowchart illustrating a method 300 with a modified step of storing the medical data.

In particular, the step 230 of storing the medical data comprises a sub-step 231 of storing the medical data itself and a sub-step 232 of further storing, in the memory cache, information suitable for identifying an original location of the medical data in the electronic medical data store ("location information") for facilitating automated updating of the medical data in the electronic medical data store with a modified version of the cached medical data.

In one example, the location information is stored in the form of a pointer or other identifying datapoint that identifies the location of the medical data in the electronic medical data store.

In another example, sub-step 232 comprises storing the medical data, in the memory cache, in the form of a same data structure as the medical data is stored in the electronic medical data store. The structure in which data is stored is indicative of its location in the original electronic medical data store, i.e., can be used to identify the location from where the data originated. This approach provides a simple and intuitive mechanism for storing location data. In this approach, sub-steps 231 and 232 are effectively combined.

Fig. 4 is a flowchart illustrating a computer-implemented method 400 according to further embodiment.

The method 400 comprises performing any method 200, 300 previously described.

The method 400 also comprises a step 410 of updating the cached medical data responsive to one or more input signals. The one or more input signals may, for instance, be received from a user interface and/or another processing system (e.g., a patient monitor or the like).

The method 400 also comprises a step 420 of updating, responsive to a user input, the medical data stored in the electronic medical data store with the updated cached medical data. The user input is received at a user interface. This method ensures that the medical data at the memory cache is only updated with the (updated) cached medical data when approved by a user.

Thus, step 420 may comprise a step 421 of determining whether or not a user input has been received that indicates that the medical data is to be updated in the electronic medical data store, and a step 422 of updating the medical data in the electronic medical data store.

Step 420 (specifically step 422) may comprise overwriting and/or appending/supplementing the medical data stored in the electronic medical data store.

In preferred examples, method 400 may comprise preventing the updated cached medical data from updating the medical data in the electronic medical data store until a user input (approving the update) is received.

The proposed method also avoids or reduces a chance that an electronic medical data store will be erroneously updated and/or reduces a number of update communications to the electronic medical data store from the memory cache. This saves processing power.

Turning back to Fig. 1, any herein described method may be carried out by the processing system 140.

In some examples, the processing system 140 is remote from the electronic medical data store 110, e.g., in a different location. Thus, a latency between the processing system and the electronic medical data store may be greater than 100ms, e.g., greater than 250ms.

In other examples, the processing system 140 is at a same location as the medical record 110. Thus, a latency between the system storing the electronic medical data store and the system performing the proposed method is preferably less than 100ms, e.g., less than 50ms.

In particular, the computer-implemented method may be performed by a medical record system that comprises the electronic medical data store. In this way, remote control over the data stored in the memory cache can be achieved. This may be advantageous to take account of, for instance, the greater processing power of a medical record system and/or to facilitate use of additional information available to the medical record system (e.g., free storage space, processing demand and so on).

There is also provided a caching system, which comprises the memory cache 120 and the processing system 140.

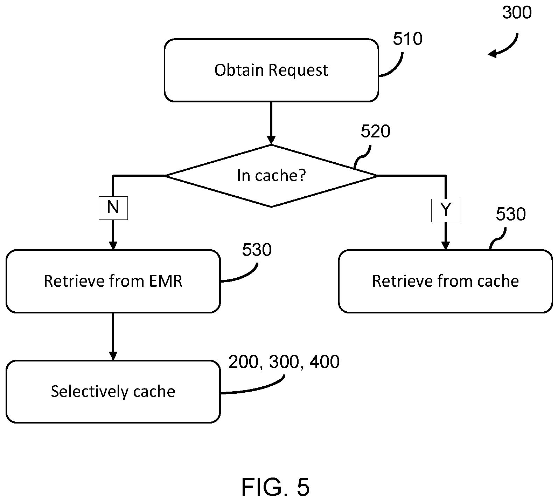

Fig. 5 is a flowchart illustrating another computer-implemented method 500 according to an embodiment. The method 500 is for retrieving medical data available for retrieval in an electronic medical data store.

The computer-implemented method comprises a step 510 of obtaining a request for the medical data and a step 520 of determining whether or not the medical data is stored in a memory cache.

The computer-implemented method 500 also comprises a step 530 of, responsive to determining that the medical data is stored in the memory cache, retrieving the medical data from the memory cache.

The computer-implemented method 500 also comprise steps 540, 550 of, responsive to determining that the medical data is not stored in the memory cache: retrieving 540 the medical data from the electronic medical data store; and performing any previously described method 200, 300, 400 for selective caching of the medical data.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa. Any reference signs in the claims should not be construed as limiting the scope.

## Claims

1. A computer-implemented method for selective caching of medical data, contained in an electronic medical data store, in a memory cache that is remote from the electronic medical data store, the computer-implemented method comprising:
receiving an indication of an intended purpose or use for the medical data;
determining whether or not the medical data is to be cached responsive to the received indication of the intended purpose or use for the medical data; and
storing the medical data in the memory cache only when it is determined that the medical data is to be cached.

2. The computer-implemented method of claim 1, wherein the step of storing the medical data further comprises storing, in the memory cache, information suitable for identifying an original location of the medical data in the electronic medical data store for facilitating automated updating of the medical data in the electronic medical data store with a modified version of the cached medical data.

3. The computer-implemented method of any of claims 1 or 2, wherein the step of storing the medical data comprises storing the medical data, in the memory cache, in a same data structure as the medical data is stored in the electronic medical data store.

4. The computer-implemented method of any of claims 1 to 3, wherein the indication of an intended purpose or use for the medical data comprises a trigger signal generated during a computer-implemented protocol or program that indicates that the medical data will be required during later processing performed by the computer-implemented protocol or program.

5. The computer-implemented method of any of claims 1 to 4, wherein the step of determining whether or not the medical data is to be cached comprises:
processing the indication of an intended purpose or use to predict an access frequency of the medical data during the intended purpose or use; and
determining whether or not the medical data is to be cached further responsive to the predicted access frequency of the medical data.

6. The computer-implemented method of any of claims 1 to 5, wherein the intended purpose or use for the medical data is an intended protocol under which the medical data is to be processed.

7. The computer-implemented method of any of claims 1 to 6, wherein the step of determining whether or not the medical data is to be cached comprises:
processing the indication of an intended purpose or use to predict a size of the medical data; and
determining whether or not the medical data is to be cached further responsive to the predicted size of the medical data.

8. The computer-implemented method of any of claims 1 to 7, wherein the step of determining whether or not the medical data is to be cached is further responsive to the content of the medical data.

9. The computer-implemented method of claim 8, wherein the step of determining whether or not the medical data is to be cached comprises determining that the medical data is not to be cached responsive to the content of the medical data being data that is manually defined by a human.

10. The computer-implemented method of any of claims 1 to 9, wherein said computer-implemented method is performed by a system that is remote from the electronic medical data store.

11. The computer-implemented method of any of claims 1 to 9, wherein said computer-implemented method is performed by a medical record system that comprises the electronic medical data store.

12. The computer-implemented method of any of claims 1 to 11, further comprising:
updating the cached medical data responsive to one or more input signals; and
updating, responsive to a user input, the medical data stored in the electronic medical data store with the updated cached medical data.

13. A computer-implemented method for retrieving medical data available for retrieval in an electronic medical data store, the computer-implemented method comprising:
obtaining a request for the medical data;
determining whether or not the medical data is stored in a memory cache;
responsive to determining that the medical data is stored in the memory cache, retrieving the medical data from the memory cache; and
responsive to determining that the medical data is not stored in the memory cache:
retrieving the medical data from the electronic medical data store; and
performing the computer-implemented method of any of claims 1 to 12 for the retrieved medical data.

14. A computer program product comprising computer program code means which, when executed on a computing device having a processing system, cause the processing system to perform all of the steps of the method according to any of claims 1 to 13.

15. A caching system for selective caching of medical data, contained in an electronic medical data store, the caching system comprising:
a memory cache remote to the electronic medical data store; and
a processing system configured to:
receive an indication of an intended purpose or use for the medical data;
determine whether or not the medical data is to be cached responsive to the received indication of the intended purpose or use for the medical data; and
store the medical data in the memory cache only when it is determined that the medical data is to be cached.
